# EUROPÄISCHE PATENTSCHRIFT

(11) **EP 2 881 062 B1**
(45) Veröffentlichungstag und Bekanntmachung des Hinweises auf die Patenterteilung: **29.03.2017**
(21) Anmeldenummer: 14004080.9
(22) Anmeldetag: 03.12.2014
(51) Int. Cl.: A61L 2/26

(54) **Aufnahmewagen für Wasch- und Desinfektionsmaschinen**
Trolley for washing and disinfection machines
Chariot récepteur pour machines de lavage et de désinfection

(30) Priorität: 04.12.2013 DE 202013009880 U
(43) Veröffentlichungstag der Anmeldung: 10.06.2015
(73) Patentinhaber: Belimed AG, 6300 Zug (CH)
(72) Erfinder: Lemaire, Josef, 84478 Waldkraiburg (DE)
(74) Vertreter: Feldkamp, Rainer

(56) Entgegenhaltungen:
- WO-A1-2009/016111
- WO-A1-2009/030599
- WO-A1-2013/068822
- WO-A1-2014/132151
- DE-B3- 10 332 150
- US-A- 5 851 484

## Beschreibung

Die Erfindung bezieht sich auf einen Aufnahmewagen für Wasch- und Desinfektionsmaschinen, die einen Waschraum aufweisen, in den jeweilige Aufnahmewagen einschiebbar sind, die mit zu waschenden und desinfizierenden Gegenständen befüllbar sind, wobei die Wasch- und Desinfektionsmaschine Versorgungsanschlüsse für die Versorgung der Aufnahmewagen mit Wasch- und Desinfektionsflüssigkeit sowie gegebenenfalls mit Luft zur Trocknung der zu reinigenden Gegenstände aufweist. Die Aufnahmewagen selbst sind mit entsprechenden Kupplungseinrichtungen zur Kupplung mit den Versorgungsanschlüssen des Waschraumes versehen und weisen ebenso wie gegebenenfalls die Maschine selbst Wascharme auf, die durch das über die Versorgungsanschlüsse zugeführte Medium in Drehung versetzt werden, um die zu reinigenden Gegenstände gründlich mit diesem Medium zu beaufschlagen und zu reinigen sowie zu desinfizieren und gegebenenfalls zu trocknen.

Derartige Wasch- und Desinfektionsmaschinen werden in größerer Anzahl in Anlagen der Sterilgutversorgung sowohl in der Medizintechnik als auch in der pharmazeutischen Industrie verwendet. Zumeist sind mehrere derartige Maschinen an einer zentralen Stelle zusammengefasst, wobei Maschinen unterschiedlicher Größe verwendet werden. Entsprechend der Größe der einzelnen Maschinen werden der jeweiligen Größe der Maschine angepasste Aufnahmewagen verwendet. Die Auslastung der einzelnen Maschinen kann im laufenden Betrieb sehr unterschiedlich sein, wobei weiterhin Probleme bei Ausfällen einzelner Maschinen entstehen können.

Der vorliegenden Erfindung liegt die Aufgabe zugrunde, eine Wasch- und Desinfektionsmaschine sowie einen Aufnahmewagen hierfür zu schaffen, die bzw. der einen flexiblen Einsatz derartiger Wasch- und Desinfektionsmaschinen ermöglicht.

Diese Aufgabe wird durch die in dem Patentanspruch 1 angegebenen Merkmale gelöst.

Vorteilhafte Ausgestaltungen und Weiterbildungen der Erfindung ergeben sich aus den Unteransprüchen.

Durch die erfindungsgemäße Ausgestaltung von zumindest einem der Aufnahmewagen als Modulwagen ist die Verwendung einer Anzahl von Aufnahmewagen, die zur Verwendung in kleineren Wasch- und Desinfektionsmaschinen ausgestaltet sind, in größeren derartigen Maschinen möglich.

Der erfindungsgemäße Modulwagen weist einen Grundrahmen, der erste Kupplungsanschlüsse zur lösbaren Verbindung mit den Versorgungsleitungen der Wasch- und Desinfektionsmaschine, aufweist, einen Tragrahmen, der zur Aufnahme einer Anzahl von Aufnahmewagen von Maschinen kleinerer Größe ausgestaltet ist, eine der Anzahl der auf dem Tragrahmen anzuordnenden Aufnahmewagen entsprechende Anzahl von Zweigleitungen auf, die mit den ersten Kupplunganschlüssen verbunden sind und zu einzelnen zweiten Kupplungsanschlüssen auf dem Tragrahmen führen, die zur Verbindung mit Kupplungsanschlüssen der auf dem Tragrahmen anzuordnenden Aufnahmewagen führen, die ihrerseits vorzugsweise mit entsprechenden Wascharmen versehen sind.

Auf diese Weise kann bei Überlastung oder Ausfall der für kleinere Aufnahmewagen vorgesehenen Wasch- und Desinfektionsmaschinen eine größere Maschine gleichzeitig mehrere Aufnahmewagen der kleineren Maschine(n) aufnehmen, so dass Ausfälle kleinerer Reinigungs- und Desinfektionsmaschinen oder eine Überbeanspruchung dieser Maschinen abgefangen werden können.

Ausführungsbeispiele der Erfindung werden im Folgenden anhand der Zeichnungen noch näher erläutert.
Figur 1 zeigt eine perspektivische Ansicht eines Modulwagens zur Aufnahme von zwei Aufnahmewagen;
Figur 2 zeigt eine Stirnansicht der Ausführungsform des Modulwagens nach Figur 1;

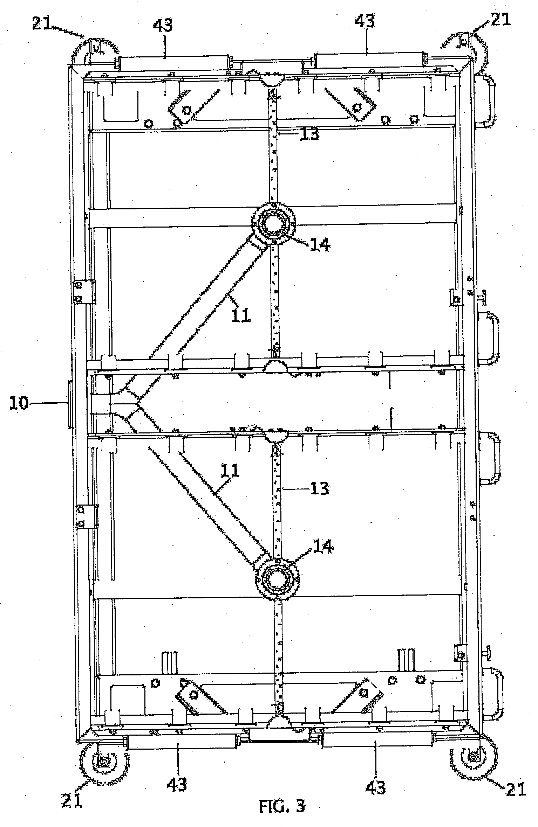

Figur 3 zeigt eine Draufsicht von oben auf den Modulwagen nach den Figuren 1 und 2;
Figur 4 zeigt eine perspektivische Ansicht des Modulwagens, der bereits mit einem Aufnahmewagen von einem Transferwagen aus übertragen wurde, während ein weiterer Transferwagen mit darauf angeordnetem Aufnahmewagen zur Übergabe bereit dargestellt ist;
Figur 5 zeigt eine weitere Ansicht, die das Ankuppeln der Transferwagen und die Überführung der Aufnahmewagen auf den Modulwagen zeigt;
Figur 6 zeigt eine Ansicht von vorne auf einen Modulwagen mit einem daran angekoppelten Transferwagen, wobei die Ankopplung der Aufnahmewagen an das Versorgungssystem des Modulwagens gezeigt ist.

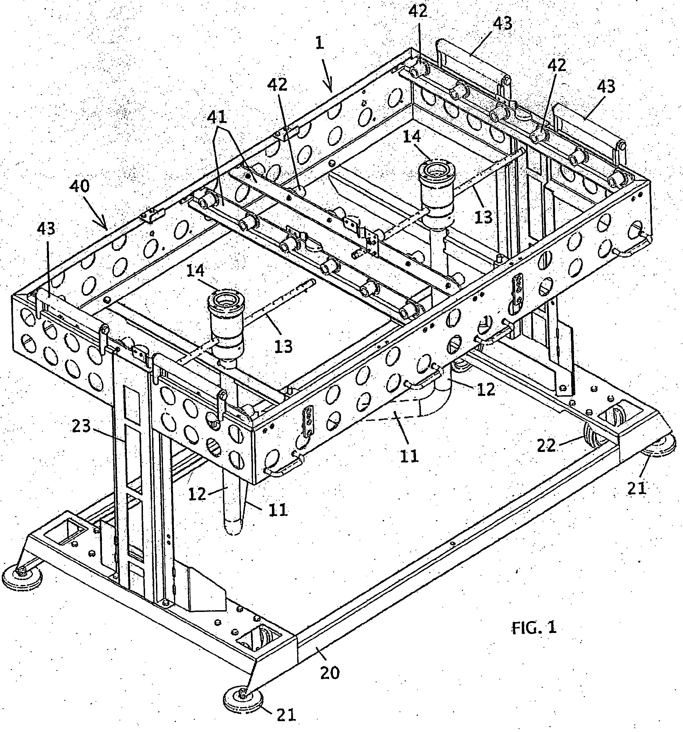

In Figur 1 ist eine perspektivische Ansicht des als Modulwagen 1 ausgebildeten Aufnahmewagens gezeigt, der einen Grundrahmen 20 aufweist, der mit Führungsrollen 21 zur Führung des Modulwagens in einer Wasch-und Desinfektionsmaschine sowie mit Tragrollen 22 versehen ist, die einen leichten Transport des Modulwagens in den Aufnahmeraum der Wasch- und Desinfektionsmaschine und aus dieser heraus ermöglichen.

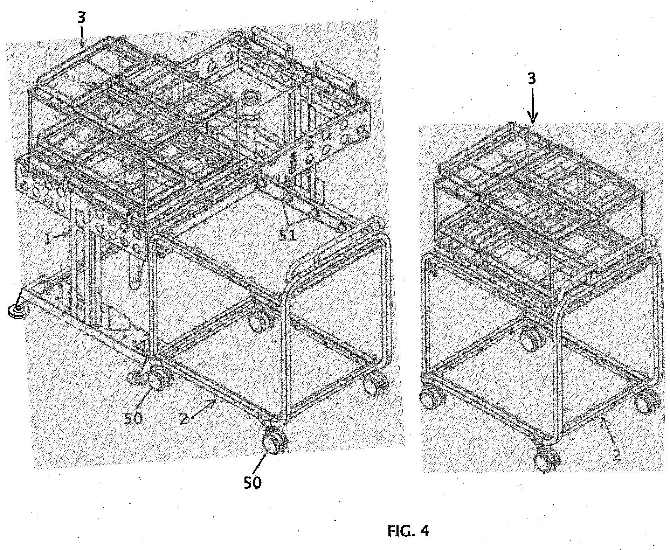

Auf dem Grundrahmen 20 ist über Ständer 23 ein Tragrahmen 40 gehaltert, der bei der dargestellten Ausführungsform zur Aufnahme von zwei Aufnahmewagen 3 in der in den Figuren 4 bis 6 dargestellten Weise dient.

Selbstverständlich ist bei anderen nicht dargestellten Ausführungsformen auch eine Ausgestaltung der Tragrahmens 40 zur Aufnahme einer anderen Anzahl von Aufnahmewagen 3 möglich.

Der Tragrahmen ist mit Handgriffen 43 versehen und weist jeweilige Führungsrollen 42 für die Aufnahmewagen 3 auf, wobei die Führungsrollen 42 an dem Außenteil des Tragrahmens 40 bzw. an an dem Tragrahmen befestigten Führungsschienen 41 drehbar gelagert sind.

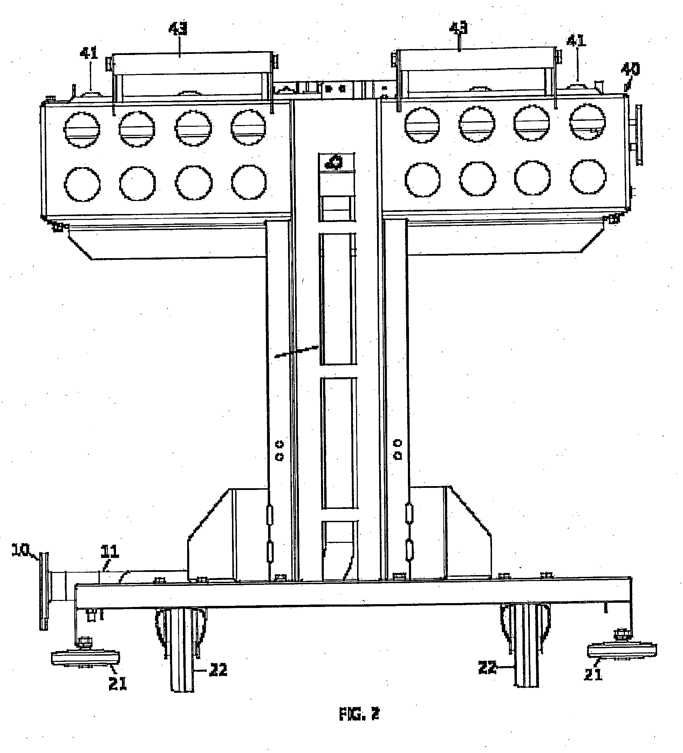

Der Modulwagen 1 ist über einen in Figur 2 dargestellten ersten Kupplungsanschluss 10 mit nicht dargestellten komplementären Kupplungsanschlüssen des Waschraumes einer Wasch- und Desinfektionsmaschine verbindbar, die beim Einschieben des Modulwagens in den Waschraum eine Verbindung mit den Versorgungsleitungen der Maschine bewirken.

Der erste Kupplungsanschluss 10 ist über Verzweigungsrohre 11 und Steigrohre 12 mit jeweiligen zweiten Kupplungsanschlüssen 14 für die Aufnahmewagen 3 verbunden.

Diese Steigrohre 12 bzw. die zweiten Kupplungsanschlüsse 14 sind weiterhin mit einer Drehlagerung für Wascharme 13 versehen, die bei Zuführung des über den ersten Kupplungsanschluss 10 zugeführten Mediums in Drehung versetzt werden und eine gleichförmige Reinigung der auf einem Aufnahmewagen 3 befindlichen Gegenstände von der Unterseite aus ermöglichen.

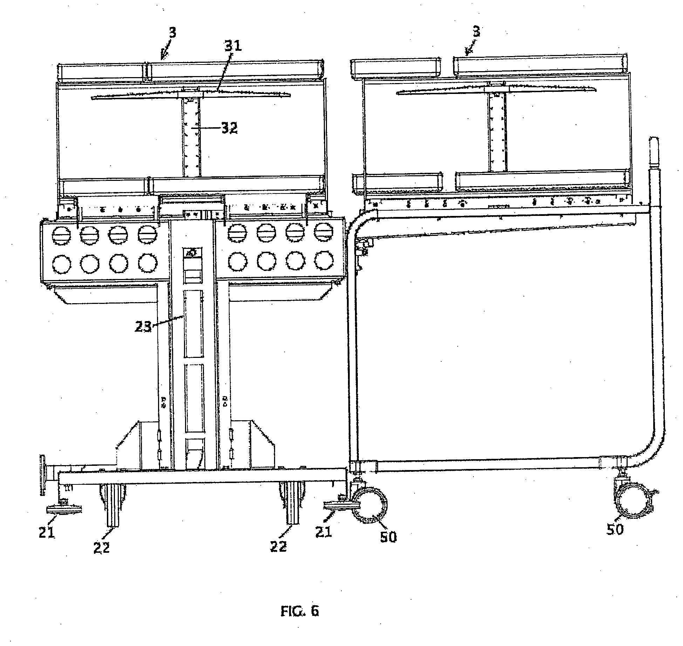

Die Aufnahmewagen 3 selbst sind, wie dies insbesondere aus Figur 6 zu erkennen ist, ihrerseits mit Steigrohren 32 versehen, die mit dem zweiten Kupplungsanschluss 14 nach dem Aufschieben der Aufnahmewagen 3 auf den Modulwagen 1 verbunden sind und ihrerseits in üblicher Weise weitere Wascharme 31 tragen, die ebenfalls durch das über das Steigrohr 32 zugeführte Medium in Drehung versetzt werden und die auf den Aufnahmewagen 3 befindlichen Gegenstände von oben reinigen.

Wie dies aus Figur 4 zu erkennen ist, können die einzelnen Aufnahmewagen 3 mit Hilfe von Transferwagen 2 auf den Modulwagen 1 überführt werden, wobei derartige Transferwagen üblicherweise zum Transport der Aufnahmewagen 3 in kleinere Wasch- und Desinfektionsmaschinen verwendet werden und damit ohnehin vorhanden sind.

Diese Transferwagen 2 sind auf Rollen 50 beweglich und tragen ihrerseits Führungsrollen 51, auf denen die einzelnen Aufnahmewagen 3 gelagert sind, wie dies in der rechten Hälfte der Figur 4 dargestellt ist.

Die Transferwagen 2 und/oder der Modulwagen 1 weisen mechanische Kupplungseinrichtungen auf, über die die Transferwagen vorübergehend starr mit dem Modulwagen 1 gekoppelt werden können, um die Aufnahmewagen 3 sicher überführen zu können.

In Figur 4 ist in der linken Hälfte ein Aufnahmewagen 3 gezeigt, der bereits von dem Transferwagen 2 auf den Modulwagen 1 überführt wurde, während die in Figur 4 rechte Hälfte des Modulwagens noch nicht mit einem Aufnahmewagen 3 belegt ist.

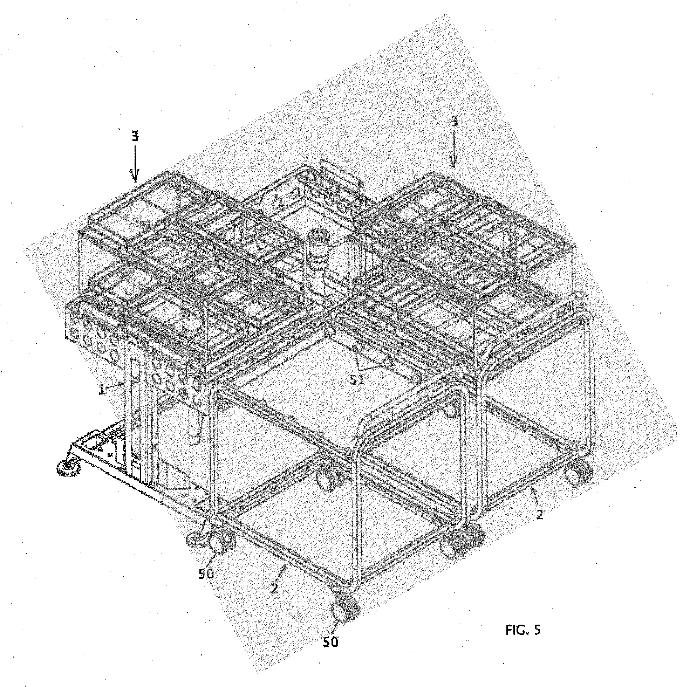

In Figur 5 ist auch der zweite Transferwagen 2 mit dem Modulwagen 1 gekoppelt, so dass ausgehend von dieser Stellung auch der zweite Aufnahmewagen 3 auf den Modulwagen 1 zu überführen ist.

In den meisten Fällen werden die Aufnahmewagen 3 von dem Transferwagen 2 direkt in Wasch- und Desinfektionsmaschinen einer ersten kleineren Größe überführt, wobei die Aufnahmewagen 3 einzeln in die kleinere Wasch- und Desinfektionsmaschine eingesetzt werden. Falls diese kleineren Wasch- und Desinfektionsmaschinen einer Sterilgut-Aufbereitungsanlage überlastet sind oder ausgefallen sind, können mit dem gleichen Transferwagen 2 die Aufnahmewagen auf einen der Modulwagen 1 überführt werden, der zur Aufnahme in einer Wasch-und Desinfektionsmaschine einer zweiten größeren Abmessung ausgebildet ist. Damit können Kapazitätsengpässe bei den kleineren Wasch- und Desinfektionsmaschinen abgefangen werden.

## Patentansprüche

1. Aufnahmewagen (1) für Wasch- und Desinfektionsmaschinen zur Reinigung von medizinischen und/oder pharmazeutischen Gegenständen, mit einem Waschraum, in den jeweilige Aufnahmewagen (1) einer ersten Größe einschiebbar sind, die mit zu waschenden und desinfizierenden Gegenständen befüllbar und über erste Kupplungsanschlüsse (10) mit Versorgungsleitungen des Waschraumes der Wasch- und Desinfektionsmaschine für Reinigungs-, Desinfektions- und Trocknungsmedien verbindbar sind,
**dadurch gekennzeichnet, dass** zumindest ein Aufnahmewagen der ersten Größe als Modulwagen (1) zur Aufnahme einer Anzahl von eine zweite Größe aufweisenden Aufnahmewagen (3) ausgebildet ist, die kleiner als die erste Größe ist, und dass der Modulwagen (1) andererseits zweite zur Kupplung mit Kupplungsanschlüssen der einzelnen kleineren Aufnahmewagen (3) ausgebildete Kupplungsanschlüsse aufweist, wobei die erste Kupplungseinrichtung (10) über Verzweigungsrohre (11) und Steigrohre (12) mit den jeweiligen zweiten Kupplungseinrichtungen (14) für die Aufnahmewagen (3) verbunden ist.

2. Aufnahmewagen nach Anspruch 1, **dadurch gekennzeichnet, dass** der als Modulwagen (1) ausgebildete Aufnahmewagen mechanische Kupplungseinrichtungen aufweist, die zur starren_Kupplung mit Transferwagen (2) ausgebildet sind, die zur Aufnahme der kleineren Aufnahmewagen (3) und deren Überführung in kleinere Wasch- und Desinfektionsmaschinen bestimmt sind und die Überführung der kleineren Aufnahmewagen auf den Modulwagen (1) ermöglichen.

3. Aufnahmewagen nach Anspruch 1 oder 2, **dadurch gekennzeichnet, dass** der Modulwagen (1) mit Kupplungs- und Verzweigungsanschlüssen (10, 11, 12, 14) zur Verbindung mit einer vorbestimmten Anzahl von Aufnahmewagen (3) versehen ist.

4. Aufnahmewagen nach Anspruch 2 oder 3, **dadurch gekennzeichnet, dass** der Modulwagen (1) mit einem auf Rollen (22) laufenden Grundrahmen (20) und einen auf diesem über Ständer (23) abgestützten Tragrahmen versehen ist, dessen Höhe der Höhe eines Tragrahmens der Transferwagen (2) entspricht.

5. Aufnahmewagen nach einem der vorhergehenden Ansprüche, **dadurch gekennzeichnet, dass** der Modulwagen (1) eine von den ersten Kupplungsanschlüssen (10) ausgehende Anzahl von Zweigleitungen aufweist, die in vertikale Steigleitungen (12) münden, die an ihrem Ende mit den zweiten Kupplungsanschlüssen zur Kupplung mit den Kupplungsanschlüssen der einzelnen Aufnahmewagen versehen sind, und dass die zweiten Kupplungsanschlüsse mit einem Drehlager zur Lagerung von Wascharmen (13) zur Reinigung der Aufnahmewagen (3) von der Unterseite aus versehen sind.

6. Aufnahmewagen nach einem der vorhergehenden Ansprüche, **dadurch gekennzeichnet, dass** der Grundrahmen (20) und/oder der Tragrahmen (40) des Modulwagens mit mechanischen Kupplungseinrichtungen zur Kupplung des Modulwagens mit Transferwagen zur Überführung jeweiliger Aufnahmewagen (3) auf den Modulwagen (1) versehen ist.

7. Aufnahmewagen nach einem der vorhergehenden Ansprüche, **dadurch gekennzeichnet, dass** die zur Aufnahme auf den Modulwagen (1) bestimmten Aufnahmewagen (3) mit Steigrohren (32) versehen sind, die mit den zweiten Kupplungsanschlüssen (14) des Modulwagens beim Aufschieben des Aufnahmewagens (3) auf den Modulwagen kuppelbar sind und ihrerseits Drehlager für Wascharme (31) tragen.

## Claims

1. Trolley (1) for washing and disinfection machines for cleaning medical and/or pharmaceutical objects, with a washing chamber into which respective trolleys (1) of a first size can be inserted, which can be filled with objects to be washed and disinfected, and which can be connected via first coupling connections (10) to supply lines of the washing chamber of the washing and disinfection machine for cleaning, disinfection and drying media, **characterized in that** at least one trolley of the first size is configured as a module trolley (1) for receiving a number of trolleys (3) having a second size, which is smaller than the first size and that the module trolley (1) on the other hand has second coupling connections configured for coupling with coupling connections of the individual smaller trolleys (3), wherein the first coupling device (10) is connected via branch pipes (11) and riser pipes (12) to the respective second coupling devices (14) for the trolleys (3).

2. The trolley according to claim 1, **characterized in that** the trolley configured as module trolley (1) has mechanical coupling devices which are configured for rigid coupling to transfer trolleys (2), which are intended for receiving the smaller trolleys (3) and transferring them into smaller washing and disinfection machines and enable the transfer of the smaller trolleys onto the module trolleys (1).

3. The trolley according to claim 1 or 2, **characterized in that** the module trolley (1) is provided with coupling and branch connections (10, 11, 12, 14) for connection to a predetermined number of trolleys (3).

4. The trolley according to claim 2 or 3, **characterized in that** the module trolley (1) is provided with a base frame (20) running on rollers (22) and a supporting frame supported thereon by means of stands (23), the height of which corresponds to the height of a supporting frame of the transfer trolley (2).

5. The trolley according to any one of the preceding claims, **characterized in that** the module trolley (1) has a number of branch lines starting from the first coupling connections (10), which open into vertical riser pipes (12) which are provided at the end thereof with the second coupling connections for coupling with the coupling connections of the individual trolleys and that the second coupling connections are provided with a pivot bearing for mounting of washing arms (13) for cleaning the trolleys (3) from the underside.

6. The trolley according to any one of the preceding claims, **characterized in that** the base frame (20) and/or the supporting frame (40) of the module trolley is provided with mechanical coupling devices for coupling the module trolley to transfer trolleys for transferring respective trolleys (3) onto the module trolley (1).

7. The trolley according to any one of the preceding claims, **characterized in that** the trolleys (3) intended for receiving on the module trolleys (1) are provided with riser pipes (32) which can be coupled to the second coupling connections (14) of the module trolley when pushing the trolley (3) onto the module trolley and for its part have pivot bearings for washing arms (31).

## Revendications

1. Chariot récepteur (1) pour des machines de lavage et de désinfection, destinées à nettoyer des objets médicaux et/ou pharmaceutiques, avec un espace de lavage dans lequel sont insérables des chariots récepteurs (1) d'une première taille qui peuvent être remplis avec des objets à laver et à désinfecter et qui, par l'intermédiaire de premiers raccords d'accouplement (10) peuvent être reliés avec des conduits d'alimentation de l'espace de lavage de la machine de lavage et de désinfection pour des agents nettoyants, désinfectants et séchants,
**caractérisé en ce qu'**au moins un chariot récepteur de la première taille est conçu sous la forme d'un chariot modulaire (1) destiné à recevoir un nombre de chariots récepteurs (3) présentant une deuxième taille qui est inférieure à la première taille et **en ce que** le chariot modulaire (1) comporte d'autre part des deuxièmes raccords d'accouplement conçus pour être accouplés avec des raccords d'accouplement de chacun des chariots récepteurs (3) plus petits, le premier système d'accouplement (10) étant relié par l'intermédiaire de conduits de ramification (11) et de conduits ascendants (12) avec les deuxièmes systèmes d'accouplement (14) respectifs pour les chariots récepteurs (3).

2. Chariot récepteur selon la revendication 1, **caractérisé en ce que** le chariot récepteur conçu en tant que chariot modulaire (1) comporte des systèmes d'accouplement mécaniques qui sont conçus pour l'accouplement rigide avec des chariots de transfert (2) qui sont destinés à recevoir les plus petits chariots récepteurs (3) et leur transfert dans des machines de lavage et de désinfection plus petites et qui permettent le transfert des plus petits chariots récepteurs sur le chariot modulaire (1).

3. Chariot récepteur selon la revendication 1 ou la revendication 2, **caractérisé en ce que** le chariot modulaire (1) est muni de raccords d'accouplement et de ramification (10, 11, 12, 14) destinés à l'assemblage avec un nombre prédéfini de chariots récepteurs (3).

4. Chariot récepteur selon la revendication 2 ou la revendication 3, **caractérisé en ce que** le chariot modulaire (1) est muni d'un cadre de base (20) se déplaçant sur roulettes (22) et un cadre porteur appuyé sur celui-ci par l'intermédiaire de supports (23) dont la hauteur correspond à la hauteur d'un cadre support du chariot de transfert (2).

5. Chariot récepteur selon l'une quelconque des revendications précédentes, **caractérisé en ce que** le chariot modulaire (1) comporte un nombre de conduits de dérivation partant des premiers raccords d'accouplement (10) qui débouchent dans des conduits ascendants (12) verticaux, qui sur leur extrémité sont munis des deuxièmes raccords d'accouplement, destinés à être accouplés avec les raccords d'accouplement de chacun des chariots récepteurs et **en ce que** les deuxièmes raccords d'accouplement sont munis d'un coussinet de pivotement pour le logement de bras de lavage (13) destinés au nettoyage des chariots récepteurs (3) à partir de la face inférieure.

6. Chariot récepteur selon l'une quelconque des revendications précédentes, **caractérisé en ce que** la cadre de base (20) et/ou le cadre porteur (40) du chariot modulaire est muni de systèmes d'accouplement mécaniques destinés à accoupler le chariot modulaire avec le chariot de transfert, pour transférer des chariots récepteurs (3) respectifs sur le chariot modulaire (1).

7. Chariot récepteur selon l'une quelconque des revendications précédentes, **caractérisé en ce que** les chariots récepteurs (3) destinés à être reçus sur le chariot modulaire (1) sont munis de tubes ascendants (32) qui peuvent être accouplés sur les deuxièmes raccords d'accouplement (14) du chariot modulaire lorsqu'on emboîte le chariot récepteur (3) sur le chariot modulaire et qui pour leur part portent des coussinets de pivotement pour des bras de lavage (1).
